# EUROPEAN PATENT APPLICATION

(11) **EP 2 335 921 A1**
(43) Date of publication of application: **22.06.2011**
(21) Application number: 10181665.0
(22) Date of filing: 28.09.2005
(51) Int. Cl.: B32B 27/28, B32B 23/08

(54) **Multi-layer films**

(30) Priority: 30.09.2004 US 614863 P
(62) Divisional of application: 05803856.3
(71) Applicant: MonoSolRX, LLC, Portage IN 46368 (US)
(72) Inventor: Fuisz, Richard C., Beverly hills, CA 902210 (US); Myers, Gary L., KINGSPORT, TN 37660 (US); Fuisz, Joseph M., Bay Harbor Island, FL 33154-1204 (US)
(74) Representative: Zeestraten, Albertus W. J.

(57) **Abstract**

The present invention relates to edible multi-layer films (10) that dissolve in water. In particular, the edible multi-layer films (10) have a first water-soluble film layer (10) and one or more additional water-soluble film layers (200) in at least partial face-to-face engagement with the first film layer (100). The film layers (100,200) include a polymer composition which contains polyethylene oxide alone or in combination with at least one water-soluble polymer. The edible multi-layer films (10) may include pockets defined between the layers that house an active component. Upon addition of water, the multi-layer film dissolves, thereby releasing the active component into the water.

## Description

### FIELD OF THE INVENTION

The present invention relates to edible multi-layer films that dissolve in water. The edible multi-layer films may contain active components for delivery into the oral cavity. Alternatively, the multi-layer films may have pockets defined between the layers that house an active component, such as, for example, powdered infant formula. Upon addition of water, the multi-layer film dissolves, thereby releasing the active component into the water.

### BACKGROUND OF THE RELATED TECHNOLOGY

It often is desirable to package drugs, food products and related consumable items into pre-determined amounts. Such consumable products conventionally are packaged in wrappers that must be removed and discarded prior to consumption. The present invention provides films that dissolve in water and are edible. Such films may be used to deliver active ingredients directly into the oral cavity, or alternatively, to package consumable products that are subsequently mixed with water. The films of the present invention dissolve in the water and the product may be consumed. The films of the present invention thereby overcome the shortcomings of the prior art.

U.S. Patent no. 5,393,528 generally discloses the use of film products for contraceptive use. The reference discloses the use of multiple layers of film, which can then be inserted into the body cavity, where, in the presence of moisture and bodily temperature, the film layers dissolve. The patent discloses the incorporation of active ingredients in the film layer composition itself, where the agent is released as the film is dissolved. The patent sets forth that the dissolvable element in the film includes a polymer, such as polyvinyl alcohol, polyethylene oxide and/or complex carbohydrates. The present invention sets forth a novel and particular composition of polymers, as well as a novel and inventive method of delivering an agent between layers of films.

### SUMMARY OF THE INVENTION

In accordance with some embodiments of the present invention, there is provided an edible multi-layer film including: a first water-soluble film layer; and one or more additional water-soluble film layers in at least partial face-to-face engagement with the first film layer, wherein said first and additional film layers comprise a polymer composition which comprises polyethylene oxide alone or in combination with at least one water-soluble polymer, and said polyethylene oxide is present in amounts of about 12.5% to about 50% by weight of said polymer.

In accordance with another embodiment, there is provided a consumable product which includes:
a) an outer container having one or more compartments;
b) one or more edible bi-layer films housed in the one or more compartments, wherein the bi-layer film includes:
   i) a first water-soluble film layer;
   ii) a second water-soluble film layer which is in at least partial face-to-face engagement with the first film layer;
   iii) one or more pockets defined between the first film layer and the second film layer; and
   iv) a food product housed in the one or more pockets, wherein the first and second film layers include a polymer composition which contains: about 20% to about 50% by weight polyethylene oxide; about 25% to about 50% by weight hydroxypropylmethyl cellulose; about 20% to about 75% by weight hydroxypropyl cellulose; and up to about 20% by weight polydextrose.

In accordance with another embodiment, there is provided a method of making an edible multi-layer film, including the steps of:
a) providing a first water-soluble film layer;
b) positioning a second water-soluble film layer in at least partial face-to-face engagement with the first film layer;
c) sealing the film layers together at the face-to-face engagement;
d) optionally positioning an additional water-soluble film layer in at least partial face-to-face engagement with the second film layer and sealing the additional layer to the second layer; and
e) repeating step d) as desired,
wherein the first, second and additional film layers include a polymer composition which contains polyethylene oxide alone or in combination with at least one water-soluble polymer.

In accordance with yet another embodiment, there is provided a method of preparing a hot liquid food product, including the steps of:
a) providing an edible multi-layer film having:
   i) a first water-soluble film layer;
   ii) one or more additional water-soluble film layers in at least partial face-to-face engagement with the first film layer;
   iii) one or more pockets defined between the first film layer and the additional film layer; and
   iv) a food product housed in the one or more pockets,
      wherein the first and the additional film layers include a polymer composition which contains polyethylene oxide alone or in combination with sodium carboxymethyl cellulose;
b) adding hot water to the multi-layer film; and
c) releasing the food product as the multi-layer film dissolves in the hot water.

In accordance with another embodiment, there is provided an edible multi-layer film including: a first water-soluble film layer; and one or more additional water-soluble film layers in at least partial face-to-face engagement with the first film layer, wherein the first and additional film layers include a polymer composition which contains a first water-soluble polymer having a first glass transition temperature and a second water-soluble polymer having a second glass transition temperature which is at least about 20°C higher than the first glass transition temperature.

In accordance with another embodiment, there is provided an edible multi-layer film including: a first water-soluble film layer; and one or more additional water-soluble film layers in at least partial face-to-face engagement with the first film layer, wherein the first and additional film layers include a polymer composition which contains a first water-soluble polymer having a melt temperature and a second water-soluble polymer having a glass transition temperature which is at least about 10°C higher than the melt temperature.

In accordance with yet another embodiment, there is provided an edible multi-layer film including: a first water-soluble film layer; and one or more additional water-soluble film layers in at least partial face-to-face engagement with the first film layer, wherein the first and additional film layers include a polymer composition which contains polyethylene oxide alone or in combination with at least one water-soluble polymer. Desirably, the multi-layer film layers of the present invention are uniform in thickness and compositional content.

### BRIEF DESCRIPTION OF THE DRAWINGS

Figure 1 is a top plan view of a bi-layer film in accordance with an embodiment of the present invention;
Figure 2 is a side elevational view of a bi-layer film in accordance with an embodiment of the present invention;
Figure 2a is a side elevational view of a multi-layer film in accordance with an embodiment of the present invention;
Figure 3 is a top plan view of a bi-layer film in accordance with another embodiment of the present invention;
Figure 4 is a cross-sectional view taken along line 4-4 of Figure 3;
Figure 5 is a cross-sectional view similar to that of Figure 4, but showing an alternative embodiment of the present invention;
Figure 6 is a cross-sectional view similar to that of Figure 4, but showing an alternative embodiment of the present invention;
Figure 7 is a top plan view of a bi-layer film in accordance with another embodiment of the present invention;
Figure 8 is a side elevational view of a baby bottle housing a bi-layer film in accordance with an embodiment of the present invention; and
Figure 9 is a side elevational view of an outer container having multiple compartments housing bi-layer films in accordance with another embodiment of the present invention.

### DETAILED DESCRIPTION OF THE INVENTION

The present invention relates to edible multi-layer films that dissolve in water. The multi-layer films may be used to deliver active ingredients directly into the oral cavity. For example, in some embodiments, the films are designed to be placed directly into the oral cavity. The user's saliva causes the edible multi-layer film to dissolve, whereby the active is released into the oral cavity. The two or more layers of the film may be the same or different, depending on the desired properties.

In other embodiments, pockets are defined between the two or more layers of the multi-layer films. These pockets may house active ingredients, such as, for example, drugs, food or powdered infant formula. Upon addition of water, the multi-layer film dissolves, thereby releasing the active ingredient contained in the pocket into the water. These multi-layer films may be housed inside compartments of an outer container for addition of water thereto.

In particular, the present invention provides edible multi-layer films that include a first water-soluble film layer and one or more additional water-soluble film layers. The two or more film layers are in at least partial face-to-face engagement with each other. One particularly desirable embodiment is a bi-layer film. Desirably, the layers are sealable or fusable to one another. In particularly desirable embodiments, the layers are heat-sealable.

In some embodiments, particularly heat-sealable embodiments, the film layers include a polymer composition that contains polymers having different melt temperatures or glass transition temperatures (softening point temperature). By including polymers having different melt or glass transition temperatures, desirable film properties, such as strength, tear resistance, flexibility, dissolution and sealing, may be varied and/or balanced.

More specifically, polymers having high glass transition temperatures provide certain desirable properties to the films, such as strength and tear resistance. The softening, or tack, point of high glass transition temperature polymers, however, may not be low enough to permit sealing at desirable temperature ranges. These polymers therefore need plasticization to seal. Conventional plasticizers may be added to such polymers to lower the glass transition temperature and permit sealing, but plasticizers tend to provide narrow sealing temperature ranges.

As such, it may be desirable to combine high glass transition temperature polymers with another polymer having a lower glass transition temperature. Polymers having low glass transition temperatures impart good sealing properties to the films. In particular, low glass transition temperature polymers melt or soften at lower temperatures. The film layers thereby become tacky enough to seal or fuse to each other at desirable temperature ranges. When combined with higher glass transition temperature polymers, the melting temperature of the overall polymer composition is lowered such that upon application of heat a seal may form to fusibly join the layers. The properties of strength and tear resistance of the higher glass transition temperature polymer also are maintained.

Otherwise, a plasticizer may be necessary to lower the glass transition temperature of the polymer composition enough to permit sealing. Plasticizers, however, as described above, provide narrow sealing ranges above which the film will melt to an undesirable extent. Control of the seal range is important, particularly when the film layers contain an active component in the pocket formed therebetween. Low glass transition temperature polymers, therefore, are desirable because they provide good sealing capabilities with broader sealing ranges. The combination of high and lower glass transition temperature polymers therefore balances the film properties of strength, tear resistance, dissolution and sealability, among others.

This provides multi-layer films that are strong enough to contain consumables or the like without tearing prior to use, yet also dissolve rapidly and almost completely when mixed with water. More specifically, in some embodiments, it is desirable to have multi-layer films that contain an active component, such as food products, that dissolve quickly and substantially or fully when mixed with water. This allows the active contents of the film to be released to form a mixture with the water. The mixture may homogenous or may require some stirring, yet provides a liquid consumable with little or no film particles remaining.

Accordingly, the polymer composition may contain at least one polymer having a low glass transition temperature, such as, for example, below 0°C, in combination with a polymer having a higher glass transition temperature. The higher glass transition temperature polymer may be about 20°C higher, more desirably about 50°C higher, and in some embodiments about 150°C higher than the first polymer.

In other embodiments, the first polymer has a melt temperature which is at least 10°C lower than the glass transition temperature of the high glass transition temperature polymer.

In view of the above, some embodiments of the present invention may include polyethylene oxide in the polymer composition, which has a low glass transition temperature. Polyethylene oxide's glass transition temperature is below 0°C. Desirably, polyethylene oxide has a glass transition temperature of about -30°C. In addition, polyethylene oxide has a melt temperature range of about 65-70°C. As such, polyethylene oxide has low melt and glass transition temperatures, which provide good sealing capabilities to the films of the present invention.

The molecular weight of polyethylene oxide used in the films of the present invention may range from about 100,000 to about 8 million. Desirably, the molecular weight of polyethylene oxide ranges from about 100,000 to about 900,000. In addition, blends of different molecular weight polyethylene oxides may be employed, as described in Applicants' co-pending U.S. Application No. 10/856,176, filed on May 28, 2004, the contents of which are incorporated herein by reference.

Polyethylene oxide may be used alone or in combination with a water-soluble polymer having a higher glass transition temperature, such as, but not limited to, water-soluble cellulosic polymers. Although it is not desirable to use such cellulosic polymers alone because they need plasticization to seal, in combination with certain other polymers such as polyethylene oxide they provide good strength, tear resistance and sealing capabilities. In particular, polyethylene oxide acts as a polymeric plasticizer in these films. It provides a low melt or glass transition temperature to the polymer composition, which offsets the higher glass transition temperature of the cellulosic polymer. The combination allows the film layers to become tacky enough to seal. Therefore, it is desirable to combine polyethylene oxide with other water-soluble polymers.

Particularly suitable cellulosic polymers are hydroxypropylmethyl cellulose, hydroxypropyl cellulose and carboxymethyl cellulose. Hydroxypropylmethyl cellulose has a glass transition temperature of about 160°C, +/- 10°C. Hydroxypropylmethyl cellulose thereby provides strength and tear resistance to the films. Hydroxypropyl cellulose has a softening point range of about 100-150°C. Carboxymethyl cellulose has neither a melt nor a glass transition temperature but degrades starting at about 227°C. The cellulosic polymers may be incorporated into the film alone or in combination with each other. Another suitable water-soluble polymer is polydextrose.

As described above, in some embodiments polyethylene oxide may be used in combination with one or both of hydroxypropylmethyl cellulose and hydroxypropyl cellulose. Polyethylene oxide may be present in amounts of about 20% to about 50% by weight of the polymer composition. Hydroxypropylmethyl cellulose may be present in amounts of about 25% to about 50% by weight of the polymer composition and/or hydroxypropyl cellulose may be present in amounts of about 20% to about 75% by weight of the polymer composition. Such films may be free of added plasticizers as the low glass transition temperature of polyethylene oxide, and to some extent hydroxypropyl cellulose, provides both flexibility and good sealing properties.

In some embodiments of the present invention, it may be desirable to add a plasticizer to lower the melting temperature of the films. The incorporation of a plasticizer in amounts of up to about 20% by weight of the polymer compositions allows for lesser amounts of plasticizing polymers such as polyethylene oxide while still enabling the films to seal. In such embodiments, polyethylene oxide may be present in amounts of about 12.5% to about 50% by weight of the polymer composition. Hydroxypropylmethyl cellulose may be present in amounts of about 25% to about 75% by weight and hydroxypropyl cellulose may be present in amounts of about 12.5% to about 75% by weight of the polymer composition.

In some embodiments of the present invention, the polymer composition contains polyethylene oxide and sodium carboxymethyl cellulose. In such embodiments, polyethylene oxide may be present in amounts of about 25% up to about 100% by weight of the polymer composition, and sodium carboxymethyl cellulose may be present in amounts of greater than 0% up to about 75% by weight of the polymer composition. More desirably, in such embodiments polyethylene oxide is present in amounts of about 50% to about 75% and sodium carboxymethyl cellulose is present in amounts of about 25% to about 50% by weight of the polymer composition.

The multi-layer films described herein dissolve when mixed with room temperature or cold water, i.e., less than about 50°C. Some embodiments of the present invention also dissolve when mixed with hot water, e.g., more than about 50°C, particularly about 70-80°C. These films dissolve much more rapidly in hot water than cold water systems.

More specifically, films containing hydroxypropylmethyl cellulose and hydroxypropyl cellulose typically dissolve in room temperature or cold water. Because these polymers gel when mixed with hot water, they are substantially less soluble therein. Films of the present invention that contain polyethylene oxide, however, dissolve in both room temperature/cold and hot water systems. In addition, sodium carboxymethyl cellulose may be used to form room temperature/cold and hot water dissolving films. Unlike hydroxypropylmethyl cellulose and hydroxypropyl cellulose, polyethylene oxide and sodium carboxymethyl cellulose films do not gel in hot water. Such films dissolve even more rapidly in hot water than cold water. Such hot water dissolving films may be particularly desirable for food products, such as hot beverages and soups, as well as for sleep medications, cough-cold preparations and the like.

For example, films having polymer compositions of polyethylene oxide alone or in combination with sodium carboxymethyl cellulose dissolve in about 20-30 seconds in cold water, but less than 20 seconds and in many cases less than 10 seconds in hot water, e.g. about 70-80°C.

It also may be desirable to add polydextrose to the films of the present invention. Polydextrose is a water-soluble polymer that serves as a filler and solubility enhancer, i.e., it increases the dissolution time of the films, without compromising the sealing properties of the films. Polydextrose may be present in amounts of up to about 40% by weight of the polymer composition, more desirably up to about 20% by weight.

In some embodiments of the present invention, the two or more film layers that form the multi-layer film are compositionally the same. Each film layer contains the same polymer composition and any optional ingredients.

In other embodiments, the two or more film layers may be different. The layers may compositionally differ in any manner, such as, different polymers, actives, flavors or other optional ingredients.

For example, a film that effervesces when placed in the mouth may be provided by incorporating an edible acid into one film layer or film pocket and a base into the other film layer or film pocket. When the film is consumed, the saliva causes the film to dissolve and the acid and base react to produce effervescence. Alternatively, the acid and base may be separated by a coating and present in a single layer. Suitable edible acids include, but are not limited to, citric acid, phosphoric acid, tartaric acid, malic acid, ascorbic acid and combinations thereof. Suitable bases include, but are not limited to, alkali metal carbonates, alkali metal bicarbonates, alkaline earth metal carbonates, alkaline earth metal bicarbonates and combinations thereof.

The layers also may differ physically, such as different sizes, shapes or thicknesses. For example, the film layers may be round, square or rectangular. Film layers of different thicknesses may be used to create a controlled release multi-layer film. Controlled-release films are more fully described in Applicants' co-pending U.S. Patent Application No. 10/074,272, filed February 14, 2002, which is incorporated herein by reference in its entirety.

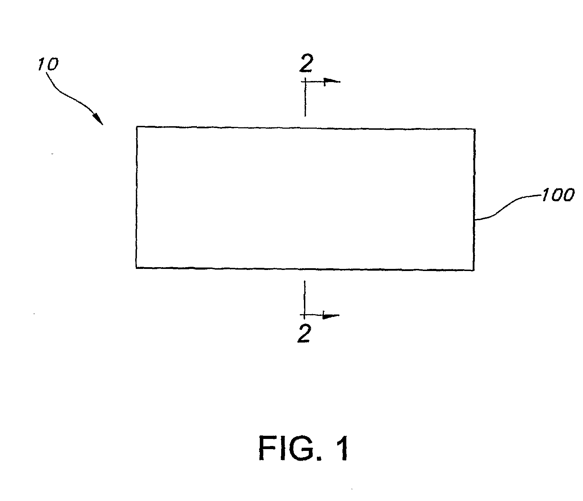

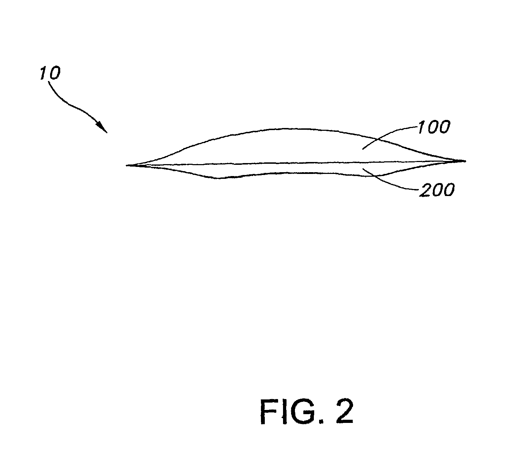

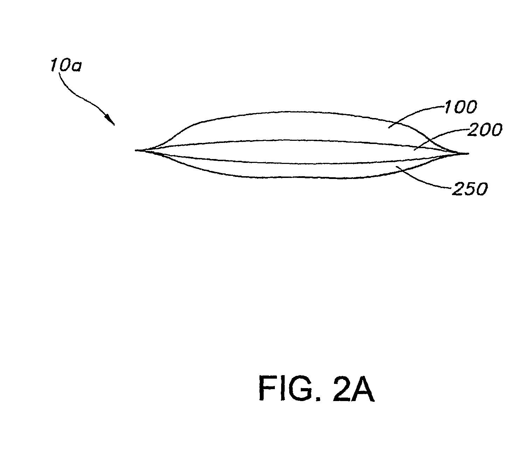

As described above, the multi-layer films include two or more film layers that may be the same or different. In some bi-layer embodiments, as depicted in Figs. 1 and 2, the film 10 has a first film layer 100 and a second film layer 200. The film layers 100 and 200 are in full face-to-face engagement with each other, as shown in Fig. 2. In some embodiments, the multi-layer film has more than two layers, such as the three-layer film depicted in Fig. 2a.

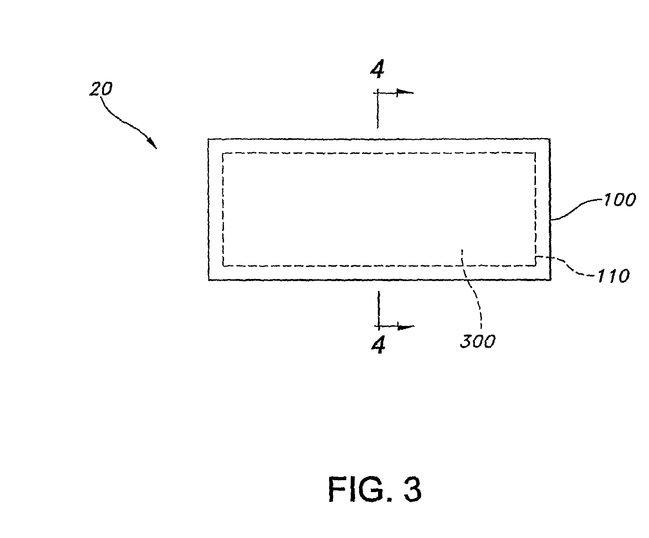

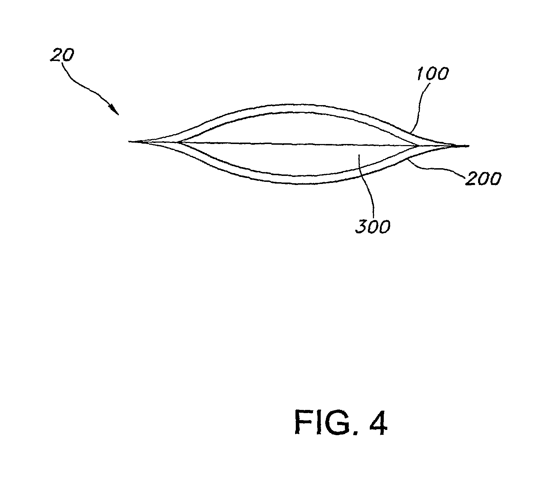

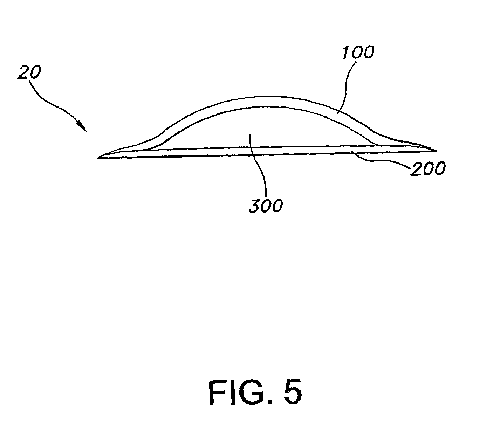

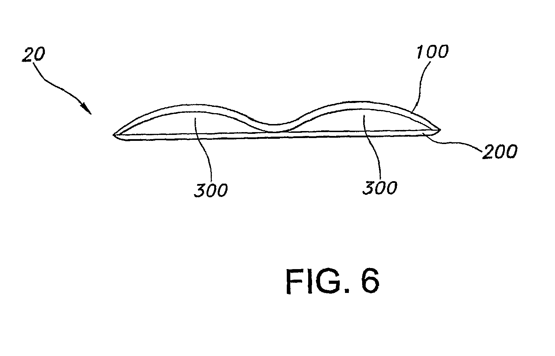

In other embodiments of the present invention, as shown in Figs. 3, 4 and 5, the first and second film layers 100 and 200 are in partial face-to-face engagement with each other. The partial face-to-face engagement may be perimetric to the film 20. The film layers may be joined, or laminated, at the perimetric engagement. A pocket 300 is thereby defined between film layers 100 and 200, as seen in Figs. 4 and 5. Alternatively, as shown in Fig. 6, multiple pockets may be formed between the film layers 100 and 200. An active component may be housed within the one or more pockets 300 for release upon dissolution of the multi-layer film.

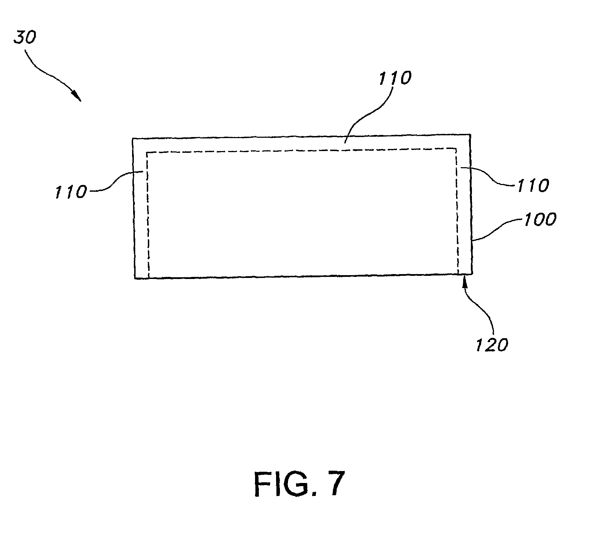

In another embodiment, the film 30 may be a single film folded over upon itself to form a bi-layer film having layers 100 and 200, as shown in Fig. 7. As in the embodiment described above, the two film layers 100 and 200 may define a pocket therebetween, which may house an active component. The film layers 100 and 200 may be joined on three sides at the point of face-to-face engagement 110 with the fold 120 forming the fourth side, as depicted in Fig. 7.

In yet another embodiment, the film layers may be gathered and pleated to form a generally spherical or cylindrical shape, such as a pouch or tube. The film layers may be joined, or sealed together, at the point of gathering to close off the opening and form a sealed enclosure.

In accordance with the present invention, the film layers may be joined at the point of their at least partial face-to-face engagement. The film layers may be joined in any manner known to those skilled in the art. For instance, the film layers may be laminated together using heat and/or pressure to seal the layers. The incorporation of a polymer having a low glass transition temperature is desirable for heat sealing the film layers together as it softens at a low temperature.

Alternatively, the film layers may be adhesively or solvent bonded together independent of the glass transition temperature of the polymer composition.

The film layers may be sealed in any shape, such as squared or rounded edges, among others. In some embodiments, the point of engagement, i.e., the fusion or sealing area, is judiciously chosen to be minimized as such lamination creates a greater film thickness and potentially slower dissolution time. Additionally, bunching and/or densification of film may occur, particularly in certain shapes, such as sharp-edged shapes, which may be slower dissolving at those lamination areas. As such, rounded edges may be desired in some embodiments to limit the amount of lamination area and speed the dissolution time and rate. Dissolution time, of course, also is related to the compositional and physical characteristics of the film, the solvent medium, the actives used, and the temperature at which the film is being dissolved, among others.

The active components housed within the film pockets include, without limitation, food products, pharmaceutical and cosmetic actives, drugs, medicaments, antigens or allergens such as ragweed pollen, spores, microorganisms, seeds, mouthwash components, flavors, fragrances, enzymes, preservatives, sweetening agents, colorants, spices, vitamins and supplements and combinations thereof. Suitable active ingredients are more fully described in Applicants' co-pending U.S. Application Nos. 10/074,272, filed February 14, 2002, 10/768,809, filed January 30, 2004, and 10/856,176, filed May 28, 2004, which are incorporated herein by reference in their entirety.

In some embodiments, the active component may be particulate, such as a powder. Examples of suitable powdered actives include food products, such as beverages and soups, among others, and infant formula. When mixed with water, the multi-layer film dissolves and the powdered active is released into the water and reconstituted into a liquid form.

Infant formula generally contains fat, carbohydrate and protein components, as well as other optional components, such as vitamins and minerals, as described in U.S. Patent Nos. 6,099,871, 6,436,464, 6,077,558, 5,422,127, 5,589,357, 5,405,637, 6,294,206, 6,472,003, 6,495,599, 6,589,576, 6,596,302, all of which are incorporated herein by reference in their entirety. Examples of suitable powdered infant formulas are those products sold under the names ENFAMIL (manufactured by Mead Johnson) and SIMILAC (manufactured by Abbott Laboratories).

In some embodiments of the present invention, it may be desirable to incorporate active components, as described above, into the film layers themselves. The actives may be incorporated into the film matrix as the film layers are prepared, which process is described more fully in U.S. Application Nos. 10,074,272, 10/768,809 and 10/856,176, referred to above. The active in the film layer(s) may be the same as or different from the active contained in the pocket(s) of the multi-layer film.
A variety of optional components also may be incorporated into the film layers, as described in U.S. Application Nos. 10,074,272, 10/768,809 and 10/856,176, referred to above. These may include, without limitation, anti-foaming agents, pigments, coloring agents, sweetening agents and flavoring agents, among others.

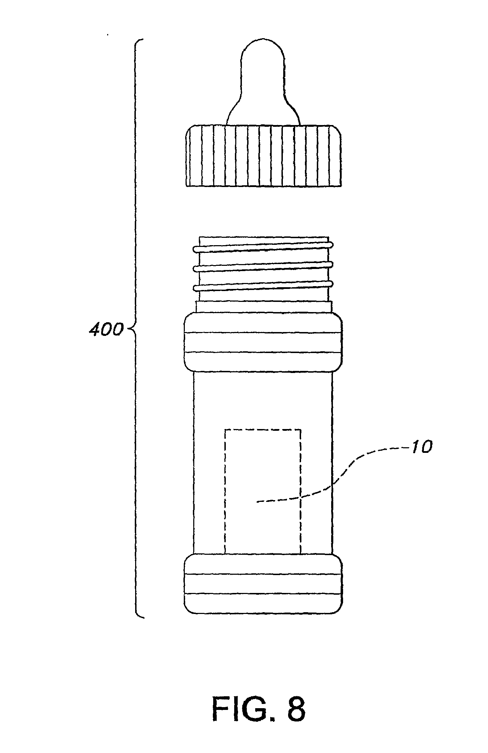

The multi-layer films of the present invention may be housed in an outer container. More specifically, the outer container may have one or more compartments, of any shape or size, in which the multi-layer film is contained. For instance, in the case of multi-layer films including infant formula, the outer container may be a disposable or reusable baby bottle 400 housing any of the films described herein, as shown in Fig. 8. The baby bottle may be any conventional baby bottle or it may be formed from a disposable plastic bag or the like.

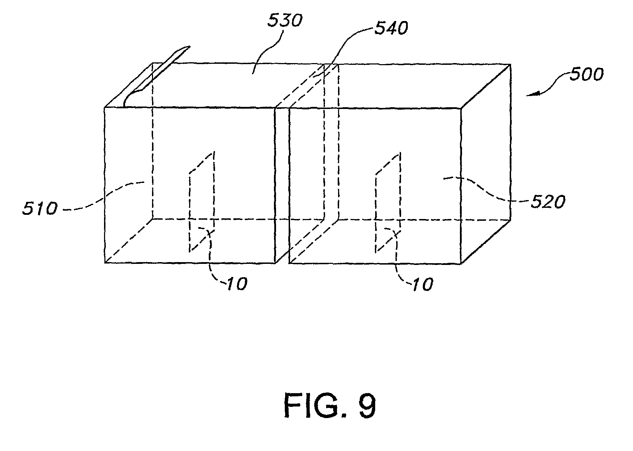

The outer container 500 may include multiple compartments 510 and 520, as shown in Fig. 9, which house a plurality of multi-layer films. As depicted in Fig. 9, the outer container 500 may have a lid 530. The lid 530 may seal the container prior to use, which then may be pulled back for opening. The outer container 500 also may be adapted for separation of the compartments 510 and 520. For instance, the container may be perforated at the point separating the compartments 540.

In some embodiments, the outer container may be another multi-layer film of the present invention. In such embodiments, one edible film houses another edible film.

Accordingly, some embodiments of the present invention are directed to a consumable product which includes an outer container, as described above, housing one or more multi-layer films of the present invention. The multi-layer films may contain a food product, such as, but not limited to, infant formula, nutritional and dietary supplements, weightloss products and nutraceutical products, among others.

The present invention also is directed to methods of making the edible multi-layer films. In particular, a first water-soluble film layer, as described above, is provided. One or more additional water-soluble film layers, which are the same as or different from the first, are positioned in at least partial face-to-face engagement with the first layer. The first and additional layers are sealed together at the face-to-face engagement. Desirably, a heat seal is formed, optionally with the use of pressure.

When the layers are in full face-to-face engagement, they may be fully laminated together to form a multi-layer film.

When the layers are in partial face-to-face engagement at the perimeters of the film layers, the layers may be perimetrically sealed together, and in addition may also have sealed sections internal to the perimeter, such as in the case of a multi-pocket embodiment. A pocket is thereby defined between the film layers. In some embodiments, an active is applied to the first film layer prior to positioning the additional film layer on the first layer. In multi-pocket embodiments, different actives may be contained in the different pockets. These actives may dissolve at different times or conditions, e.g., different temperatures or pH.

The active may be in the form of a powder, which may be sprinkled onto the first film layer or a coating that may be applied by spraying or brushing thereon. Once the additional film layer is added, the layers are sealed together, thereby housing the active in the pocket between the layers. Additional film layers may then be added in a similar manner.

More specifically, the first film layer may be provided over a mold, which has a plurality of cavities in the desired shape of the final film product. A vacuum may be applied to the first film layer positioned in the cavities. Subsequently, the active component may be added to the cavities, and then the additional film layer may be added to the top. Heat and/or pressure may be applied to seal the film layers together at the desired location.

Alternatively, a water-soluble film, as described above, is provided. The film is then folded over upon itself, thereby creating two film layers. The film layers are then sealed together at their at least partial face-to-face engagement. When the face-to-face engagement is at the perimeters of the layers, the film is thereby sealed on three sides.

### EXAMPLES

### Examples A-D:

Water-soluble film compositions of the present invention were prepared using the amounts described in Table 1.

**TABLE 1**

| **Component** | **A-D (weight in g)** |
|---|---|
| Polyethylene oxide | 17.94 |
| Hydroxypropyl cellulose | 17.94 |
| Polydextrose | 22.95 |
| Sucralose | 0.2 |
| Sodium benzoate | 0.04 |
| Glyceryl Monooleate¹ | 0.8 |
| Red coloring | 0.08 |
| Water | 120 |

| | |
|---|---|
| ¹ALDO MO K FG, available from Lonza Inc. | |

The ingredients listed in Table 1 were combined by mixing until a uniform mixture was achieved. The mixture therefore was uniform in content. The mixture was separated into compositions A, B, C and D. Composition A was 71.98g, whereas compositions B-D were each 35.99g. The following components were then added to compositions A-D in the amounts described in Table 2.

**TABLE 2**

| | **Weight (g)** | | | |
|---|---|---|---|---|
| **Component** | **A** | **B** | **C** | **D** |
| Citric acid | 1.6 | | | |
| Polydextrose | 1.53 | | | |
| Butylated hydroxytoluene | 0.032 | 0.016 | 0.016 | 0.016 |
| Taste-masking flavor | 0.96 | 0.48 | 0.48 | 0.48 |
| Cooling agent¹ | 0.7 | 0.35 | 0.35 | 0.35 |
| Wild cherry flavor | 3.2 | | | 1.6 |
| Mango flavor | | 1.6 | | |
| Tropical flavor | | | 1.6 | |
| Sodium bicarbonate | | 1.4 | 1.4 | 1.4 |
| Zinc gluconate | | 0.16 | 0.16 | 0.16 |
| Chlorine dioxide solution¹ | | 0.8 | 0.8 | 0.8 |

| | | | | |
|---|---|---|---|---|
| ¹ Combination of menthol and WS-3, available from Millenium Chemical ²2% solution containing 0.016g chlorine dioxide | | | | |

The above components for each of compositions A through D were combined by mixing until a uniform mixture was achieved, and then cast into films on release paper using a K-Control Coater with a micrometer adjustable wedge bar set at 250 microns (RK Print Coat Instruments, Ltd.). The wedge bar of the K Control Coater is an adjustable spreading blade that produces a wet film thickness equal to the gap setting. The gap setting is micrometer controlled such that films of certain uniform thicknesses can be made. Any film thickness can be chosen. In this Example, the wedge bar was set at 250 microns to create films having a uniform thickness at that level.

The films were dried for about 14 minutes at 80°C to moisture levels of about 4%. The films were cut into individual film layers (A through D) of approximately 23mm by 34mm.

Three bi-layer films were prepared from film layers A through D. The three bi-layer films were: (1) film layer A to film layer B; (2) film layer A to film layer C; and (3) film layer A to film layer D.

In particular, the film layers were laminated together using heat and very little pressure (Fuji Impulse Sealer, Model V-300). The Fuji Impulse Sealer has two opposing metal arms, or platens, which each have a flat heating tape on the metal surface. The films were placed between the opposing arms and one arm was manually brought down to meet the other arm to seal the film. As such, the films were sealed by heat and very little hand pressure, i.e., sufficient to bring the arms together to allow sealing. The sealing times and temperature for the settings of the Fuji Impulse Sealer are as follows:

| **Setting** | **Temperature (°C)** | **Time (secs)** |
|---|---|---|
| 1 | 45 | Less than 0.27 |
| 2 | 45 | 0.27 |
| 3 | 85 | 0.50 |
| 4 | 109 | 0.75 |
| 5 | 130 | 1.00 |
| 6 | 165 | 1.30 |
| 7 | 189 | 1.50 |
| 8 | 218 | 1.63 |
| 9 | 225 | 1.75 |
| 10 | 230 | 2.00 |

The three bi-layer films that were prepared contained layers that were compositionally different. The film layers could also be laminated to another layer of the same composition to form a bi-layer film having two layers that are compositionally alike.

### Example E:

A water-soluble film composition of the present invention was prepared using the following components: polyethylene oxide; hydroxypropylmethyl cellulose; polydextrose; and Vitamin C. These components were combined by mixing until a uniform mixture was achieved, and then cast into film on release paper using a K-Control Coater with a micrometer adjustable wedge bar set at 250 microns. As described above in Examples A-D, the wedge bar setting produced a film of uniform thickness. The films therefore were uniform in content and thickness.

The film was dried and cut into individual film layers (pieces) of approximately 23mm by 34mm. About 25mg of dextromethorphan HBr (60% w/w) was sprinkled on one layer of the film. Another layer of the film was placed on top of the film containing the dextromethorphan. The two film layers were laminated together with heat and very little pressure, as described above in Examples A-D (using the Fuji Impulse Sealer), thereby encapsulating the drug within the bi-layer film product.

### Examples F-AA:

Water-soluble film compositions of the present invention were prepared using the amounts described in Table 3.

**TABLE 3**

| | **Component (wt. %)** | | | | |
|---|---|---|---|---|---|
| **Composition** | **HPMC** | **PEO** | **HPC** | **Polydextrose** | **Plasticizer¹** |
| F | 100 | | | | 41.70 |
| G | 75 | 25 | | | |
| H | 50 | 50 | | | |
| I | 75 | 25 | | | 5 |
| J | 75 | 25 | | | 15 |
| K | 75 | 25 | | | 25 |
| L | 75 | 25 | | | 35 |
| M | | | 100 | | 41.70 |
| N | | 25 | 75 | | |
| O | | 50 | 50 | | |
| P | 75 | | 25 | | |
| Q | 50 | | 50 | | |
| R | 75 | 12.5 | 12.5 | | |
| S | 50 | 25 | 25 | | |
| T | 75 | | 25 | | 10 |
| U | 75 | 12.5 | 12.5 | | 10 |
| V | 50 | 25 | 25 | | 10 |
| W | 75 | 12.5 | 12.5 | | 20 |
| X | 50 | 25 | 25 | | 20 |
| Y | 40 | 20 | 20 | 20 | 10 |
| z | 25 | 25 | 50 | | |
| AA | 40 | 20 | 20 | 20 | |

| | | | | | |
|---|---|---|---|---|---|
| ¹ Mixture of propylene glycol and glycerin | | | | | |

The above components for each composition were combined by mixing until a uniform mixture was achieved, and then cast into film on release paper using a K-Control Coater with a micrometer adjustable wedge bar, as described above in Examples A-D. The bar was set at various micron settings for compositions F through AA, from 400 to 620 microns, with a specific setting for each composition. The wedge bar setting for each composition produced a film of uniform thickness. The films therefore were uniform in content and thickness.

The films were dried for about 17 minutes at 80°C to varying moisture levels. The dried films had moisture levels of about 10% or less. The films were cut into individual film pieces, or layers. Individual pieces, or layers, were sealed on one edge by application of heat and very little pressure, as described above in Examples A-D (using the Fuji Impulse Sealer). The results of the heat sealing for compositions F through AA are provided below in Table 4. In particular, Table 4 lists the temperature (or range) at which each composition sealed, or indicates otherwise if sealing did not occur.

**TABLE 4**

| **Composition** | **Heat Seal (°C)** |
|---|---|
| F | 165 |
| G | No seal |
| H | 165 |
| I | 225 |
| J | 130-189 |
| K | 130-165 |
| L | 130-165 |
| M | No seal |
| N | 109 |
| O | 85 |
| P | No seal |
| Q | No seal |
| R | No seal |
| S | 130-230 |
| T | No seal |
| U | 230 |
| V | 130-230 |
| W | 230 |
| X | 109-230 |
| Y | 109-189 |
| Z | 130 |
| AA | 109-189 |

Composition F sealed at 165°C, however, it had a slow dissolution time due, at least in part, to the absence of any polyethylene oxide and polydextrose. In particular, when placed in cold water, the bi-layer film of Composition F began to open in about 3 minutes and 10 seconds. After about 10 minutes, the film started leaking, i.e., the weak points of the film began to leak and delaminate).

The remaining compositions all had faster dissolution times, however, some compositions did not seal, as indicated in Table 4 above. In general, these compositions failed to seal because their melt or glass transition temperature was not within the temperature range of the Fuji heat sealer (about 85-230°C). This is a commercially available heat sealer, similar to other commercially available heat sealing equipment with a common temperature range. To be able to use such commercially available equipment in these temperature ranges to seal thin films and provide the appropriate level of tackiness to the films, the polymer composition needs to be balanced.

More specifically, composition G failed to seal within the tested temperature range because, at least in part, it contained predominantly HPMC (75%), which has a high glass transition temperature (about 160°C), and a much lesser amount of PEO (25%), which acts to lower the overall glass transition temperature of the polymer composition. Composition G also contained no plasticizer to assist in lowering the glass transition temperature.

Composition M is indicated as a failure to seal because it was too tacky to test. Composition M was too tacky because, at least in part, it contained 100% HPC, which has a lower glass transition temperature than HPMC, as well as a plasticizer.

Composition P failed to seal within the tested temperature range because, at least in part, similar to composition G, it contained predominantly HPMC (75%) and only 25% HPC. Composition P contained too small an amount of HPC and no PEO at all. Furthermore, composition P contained no plasticizer to lower the glass transition temperature.

Composition Q failed to seal within the tested temperature range because, at least in part, it contained only a 50%/50% blend of HPMC and HPC, and no PEO or plasticizer to lower the glass transition temperature enough to permit sealing.

Composition R failed to seal within the tested temperature range because, at least in part, it contained predominantly HPMC (75%) and not enough PEO and HPC (12.5% each) with no plasticizer. In contrast, compositions U and W, which both included the same polymer ratio (75%/12.5%/12.5%), sealed within the tested range. Compositions U and W each included a plasticizer, which lowered the glass transition temperature enough to permit sealing.

Also in contrast to composition R, compositions Z and AA both contained the same polymer combination (HPMC, PEO and HPC), however, with a lower amount of HPMC relative to the higher amounts of PEO and HPC. Neither composition contained a plasticizer, but both sealed within the tested range. PEO and H PC both have lower glass transition temperatures than HPMC, and were present in amounts sufficient to lower the melt temperature of the polymer composition such that a seal formed.

Composition T failed to seal within the tested range because, at least in part, as in composition P, it did not include any PEO. Although composition T included a low level of a plasticizer (10%), it was not enough to permit sealing without some amount of PEO in the polymer blend.

Bi-layer films were prepared from compositions Y, Z and AA containing infant formula in the pocket between the layers. The bi-layer films each were added to a baby bottle containing about 2 ounces of cold water and shaken for about 1 to 2 minutes. The resulting formulation from composition Y contained some undissolved film particles, whereas those of compositions Z and AA had significantly less undissolved particles.

### Examples AB-AH:

Water-soluble film compositions of the present invention were prepared using the polymer compositions described in Table 5.

**TABLE 5**

| | **Composition (wt. % based on polymer composition)** | | | | | | |
|---|---|---|---|---|---|---|---|
| **Component** | **AB** | **AC** | **AD** | **AE** | **AF** | **AG** | **AH** |
| Polyethylene oxide¹ | 25 | 37.5 | 50 | 75 | 100 | 80 | 60 |
| Sodium carboxymethyl cellulose² | 75 | 62.5 | 50 | 25 | | | |
| Polydextrose | | | | | | 20 | 40 |

| | | | | | | | |
|---|---|---|---|---|---|---|---|
| Solution containing 20% PEO, 79.8% water and 0.2% glyceryl monooleate ² Solution containing 10% sodium CMC, 89.87% water and 0.13% glyceryl monooleate | | | | | | | |

The above components for each composition were combined by mixing until a uniform mixture was achieved, and then cast into film on release paper using a K-Control Coater with an adjustable wedge bar, as described above in Examples A-D. The wedge bar was set at various micron settings for compositions AG through AH, from 350 to 450 microns, with a specific setting for each composition. The wedge bar setting for each composition produced a film of uniform thickness. The films therefore were uniform in content and thickness.

The films were dried for about 12-13 minutes at 80°C to varying moisture levels. The dried films had moisture levels of less than about 8%.

Films AB and AC contracted during drying and became brittle and delaminated. Films AB and AC, therefore, may have too low an amount of polyethylene oxide in the polymer composition (25% and 37.5%, respectively) when in combination with carboxymethyl cellulose. In contrast, films AD and AE, which similarly contained both polyethylene oxide and carboxymethyl cellulose, were flexible, exhibited good tear resistance and sealed to form bi-layer films. Films AD and AE included higher amounts of polyethylene oxide than AB and AC (50% and 75%, respectively).

Film AD sealed at temperatures of about 45-109°C using a Fuji Impulse Sealer. A bi-layer film including powdered KOOL-AID in the pocket between the layers was prepared. The layers were sealed at about 45°C using a Fuji Impulse Sealer. The bi-layer film containing KOOL-AID was added to a beaker containing about 74°C water. The film opened in the hot water to release the KOOL-AID in about 4 seconds and substantially or fully dissolved in less than 10 seconds.

Film AE sealed at temperatures of about 85°C using a Fuji Impulse Sealer.

Film AF (100% PEO) was flexible, exhibited good tear resistance and strength and sealed to form bi-layer films. Film AF sealed at temperatures of about 45-85°C.

Tear resistance was measured by a panel test in which members tried to tear the film apart by pulling on opposing ends of the film. Films that tore cleanly received a low grade. Films that stretched a little and began to break received a moderate grade, and films that stretched and were difficult to tear received a high grade.

Two bi-layer films of film AF including powdered KOOL-AID in the pockets between the layers were prepared. The layers were sealed at about 45°C using a Fuji Impulse Sealer. One of the bi-layer films was added to a beaker containing about 80°C water. The film opened and dissolved in the hot water to release the KOOL-AID in less than 10 seconds. The second bi-layer film was added to a beaker containing about 22°C water. The film opened and substantially or fully dissolved in the cold water in less than 20 seconds.

Films AG and AH contained polyethylene oxide and polydextrose in the polymer composition. Both films were flexible, exhibited good tear resistance and strength and sealed to form bi-layer films.

Film AG sealed at temperatures of about 45-85°C using a Fuji Impulse Sealer. Two bi-layer films including powdered KOOL-AID in the pockets between the layers were prepared. The layers were sealed between about 45 and 85°C. One of the bi-layer films was added to a beaker containing about 80°C water. The film opened and dissolved in the hot water to release the KOOL-AID in less than 10 seconds. The second bi-layer film was added to a beaker containing about 22°C water. The film opened and substantially or fully dissolved in the cold water in less than 20 seconds.

Film AH sealed at temperatures of about 60-85°C using a Fuji Impulse Sealer. Three bi-layer films were prepared. The first bi-layer film contained powdered KOOL-AID in the pocket between the layers. This bi-layer film was added to a beaker containing about 19°C water. The film opened and dissolved in the cold water to release the KOOL-AID in less than 20 seconds. The second bi-layer film contained coffee in the pocket between the layers. This bi-layer film was added to a beaker containing about 75°C water. The film opened and dissolved in the hot water to release the coffee in about 11 seconds. The third bi-layer film also contained coffee in the pocket between the layers. This bi-layer film was added to a beaker containing about 22°C water. The film opened and substantially or fully dissolved in the cold water in about 35 seconds.

**Example AI:** Bi-layer films containing coffee in the pockets between the layers were prepared. In particular, three coffee containing bi-layer films were prepared using compositions AF, AG and AH (components listed in Table 5 above). These compositions contained 0%, 20% and 40% polydextrose, respectively. The three bi-layer films were added to a beaker containing about 80-85°C water. The times required for the films to open and substantially or fully dissolve in the hot water are indicated in Table 6 below.

**TABLE 6**

| **Composition** | **Time (seconds) at 80°C** | **Time (seconds)at 22°C** |
|---|---|---|
| AF | 17.5 | 31 |
| AG | 12 | |
| AH | 14 | 35 |

As seen in the table above, addition of polydextrose to polyethylene oxide bi-layer films improves the hot water solubility without affecting sealing properties.

Two more bi-layer films containing coffee in the pockets were prepared from compositions AF and AH. The two bi-layer films were added to a beaker containing about 22°C water. The times required for the films to open and substantially or fully dissolve in the cold water are indicated in Table 6 above.

## Claims

1. An edible multi-layer film comprising:
a first water-soluble film layer; and
one or more additional water-soluble film layers in at least partial face-to-face engagement with said first film layer,
wherein said first and additional film layers comprise a polymer composition which comprises polyethylene oxide alone or in combination with at least one water-soluble polymer.

2. The multi-layer film according to claim 1, wherein said water-soluble polymer is selected from the group consisting of hydroxypropylmethyl cellulose, hydroxypropyl cellulose, sodium carboxymethyl cellulose, polydextrose and combinations thereof.

3. The multi-layer film according to claim 1, wherein said additional film layer is in full face-to-face engagement with said first film layer.

4. The multi-layer film according to claim 1, wherein said additional film layer is in perimetric face-to-face engagement with said first film layer.

5. The multi-layer film according to claim 1, wherein said first film layer and said additional film layer are joined at said at least partial face-to-face engagement.

6. The multi-layer film according to claim 5, wherein said first film layer and said additional film layer are heat sealed at said at least partial face-to-face engagement.

7. The multi-layer film according to claim 1, further comprising one or more pockets defined between said first film layer and said additional film layer.

8. The multi-layer film according to claim 7, further comprising an active component housed in said one or more pockets.

9. The multi-layer film according to claim 8, wherein said active component is selected from the group consisting of: food products; pharmaceutical agents; cosmetic agents; drugs; medicaments; antidotes; vaccines; antigens or allergens; mouthwash components; flavors; fragrances; enzymes; preservatives; sweetening agents; colorants; spices; vitamins; and combinations thereof.

10. The multi-layer film according to claim 8, wherein said active component comprises dextromethorphan.

11. The multi-layer film according to claim 8, wherein said active component comprises a powder.

12. The multi-layer film according to claim 11, wherein said active component comprises infant formula.

13. The multi-layer film according to claim 12, wherein said infant formula comprises a fat component, a carbohydrate component, and a protein component.

14. The multi-layer film according to claim 1, wherein one or both of said first film layer and said additional film layer further comprises an active component.

15. The multi-layer film according to claim 13, wherein said active component is selected from the group consisting of: pharmaceutical agents; cosmetic agents; drugs; medicaments; antidotes; vaccines; antigens or allergens; mouthwash components; flavors; fragrances; enzymes; preservatives; sweetening agents; colorants; spices; vitamins; and combinations thereof.

16. The multi-layer film according to claim 1, wherein said polyethylene oxide has a molecular weight of about 100,000 to about 900,000.

17. The multi-layer film according to claim 1, wherein said polyethylene oxide is present in amounts of about 20% to about 50% by weight of said polymer composition.

18. The multi-layer film according to claim 2, wherein said hydroxypropylmethyl cellulose is present in amounts of about 25% to about 50% by weight of said polymer composition.

19. The multi-layer film according to claim 2, wherein said hydroxypropyl cellulose is present in amounts of about 20% to about 75% by weight of said polymer composition.

20. The multi-layer film according to claim 1, wherein said first film layer and said additional film layer are free of added plasticizers.

21. The multi-layer film according to claim 1, wherein said first film layer and said additional film layer further comprise a plasticizer.

22. The multi-layer film according to claim 21, wherein said plasticizer is present in amounts of up to about 20% by weight of said polymer composition.

23. The multi-layer film according to claim 21, wherein said polyethylene oxide is present in amounts of about 12.5% to about 50% by weight of said polymer composition.

24. The multi-layer film according to claim 21, wherein said first film layer and said additional film layer further comprise hydroxypropylmethyl cellulose present in amounts of about 25% to about 75% by weight of said polymer composition.

25. The multi-layer film according to claim 21, wherein said first film layer and said additional film layer further comprise hydroxypropyl cellulose present in amounts of about 12.5% to about 75% by weight of said polymer composition.

26. The multi-layer film according to claim 1, wherein said first film layer and said additional film layer further comprise up to about 40% polydextrose by weight of said polymer composition.

27. The multi-layer film according to claim 1, wherein said first film layer is compositionally the same as said additional film layer.

28. The multi-layer film according to claim 1, wherein said first film layer is compositionally different from said additional film layer.

29. The multi-layer film according to claim 28, wherein said first film layer comprises an edible acid and said additional film layer comprises a base.

30. The multi-layer film according to claim 1, further comprising an outer container which houses said multi-layer film.

31. The multi-layer film according to claim 1, wherein said water-soluble cellulosic polymer comprises sodium carboxymethyl cellulose.

32. The multi-layer film according to claim 31, wherein said sodium carboxymethyl cellulose is present in amounts of about 25% to about 50% by weight of said polymer composition.

33. The multi-layer film according to claim 31, wherein said polyethylene oxide is present in amounts of about 50% to about 75% by weight of said polymer composition.

34. The multi-layer film according to claim 1, wherein said film comprises three-film layers.

35. A consumable product comprising:
a) an outer container having one or more compartments;
b) one or more edible bi-layer films housed in said one or more compartments, wherein said bi-layer film comprises:
i) a first water-soluble film layer;
ii) a second water-soluble film layer which is in at least partial face-to-face engagement with said first film layer;
iii) one or more pockets defined between said first film layer and said second film layer; and
iv) a food product housed in said one or more pockets,
wherein said first and second film layers comprise a polymer composition which comprises:
about 20% to about 50% by weight polyethylene oxide;
about 25% to about 50% by weight hydroxypropylmethyl cellulose;
about 20% to about 75% by weight hydroxypropyl cellulose; and
up to about 20% by weight polydextrose.

36. The consumable product according to claim 35, wherein said food product comprises infant formula.

37. A method of making an edible multi-layer film, comprising the steps of:
a) providing a first water-soluble film layer;
b) positioning a second water-soluble film layer in at least partial face-to-face engagement with the first film layer;
c) sealing the film layers together at the face-to-face engagement;
d) optionally positioning an additional water-soluble film layer in at least partial face-to-face engagement with the second film layer and sealing the additional layer to the second layer; and
e) repeating step d) as desired,
wherein said first, second and additional film layers comprise a polymer composition which comprises polyethylene oxide alone or in combination with at least one water-soluble polymer.

38. The method according to claim 37, wherein the step of sealing the film layers comprises applying heat to seal the film layers.

39. The method according to claim 37, wherein the step of sealing the film layers comprises sealing the film layers on at least three sides.

40. The method according to claim 37, wherein the step of sealing the film layers comprises perimetrically sealing the film layers.

41. The method according to claim 37, further comprising the step of applying an active component to the first film layer prior to positioning the second film layer in at least partial face-to-face engagement with the first film layer.

42. The method according to claim 41, wherein the active component comprises a food product.

43. The method according to claim 37, wherein the step of providing a first water-soluble film layer comprises positioning a first water-soluble film layer over a plurality of cavities.

44. The method according to claim 43, further comprising the step of applying a vacuum to the first water-soluble film layer positioned in the plurality of cavities.

45. A method of preparing a hot liquid food product, comprising the steps of:
a) providing an edible multi-layer film comprising:
i) a first water-soluble film layer;
ii) one or more additional water-soluble film layers in at least partial face-to-face engagement with said first film layer;
iii) one or more pockets defined between said first film layer and said additional film layer; and
iv) a food product housed in said one or more pockets,
wherein said first and said additional film layers comprise a polymer composition which comprises polyethylene oxide alone or in combination with sodium carboxymethyl cellulose;
b) adding hot water to the multi-layer film; and
c) releasing the food product as the multi-layer film dissolves in the hot water.

46. An edible multi-layer film comprising:
a first water-soluble film layer; and
one or more additional water-soluble film layers in at least partial face-to-face engagement with said first film layer,
wherein said first and additional film layers comprise a polymer composition which comprises a first water-soluble polymer having a first glass transition temperature and a second water-soluble polymer having a second glass transition temperature which is at least about 20°C higher than said first glass transition temperature.

47. The multi-layer film according to claim 46, wherein said polymer composition has a third glass transition temperature which is greater than about 30°C.

48. The multi-layer film according to claim 46, wherein said second glass transition temperature is at least about 50°C higher than said first glass transition temperature.

49. An edible multi-layer film comprising:
a first water-soluble film layer; and
one or more additional water-soluble film layers in at least partial face-to-face engagement with said first film layer,
wherein said first and additional film layers comprise a polymer composition which comprises a first water-soluble polymer having a melt temperature and a second water-soluble polymer having a glass transition temperature which is at least about 10°C higher than said melt temperature.

50. An edible multi-layer film comprising:
a first water-soluble film layer; and
one or more additional water-soluble film layers in at least partial face-to-face engagement with said first film layer,
wherein said first and additional film layers comprise a polymer composition which comprises polyethylene oxide alone or in combination with at least one water-soluble polymer and wherein said multi-layer film layers are uniform in thickness and compositional content.
